# NEUE EUROPÄISCHE PATENTSCHRIFT

(11) **EP 1 210 386 B2**
(45) Veröffentlichungstag und Bekanntmachung des Hinweises auf die Entscheidung über den Einspruch: **29.10.2014**
(45) Hinweis auf die Patenterteilung: 07.07.2004
(21) Anmeldenummer: 00954384.4
(22) Anmeldetag: 28.07.2000
(51) Int. Cl.: C08J 7/06, C08K 3/08, A61L 29/04

(54) **VERFAHREN ZUR HERSTELLUNG VON ANTIMIKROBIELLEN KUNSTSTOFFKÖRPERN MIT VERBESSERTEM LANGZEITVERHALTEN**
METHOD OF PRODUCING ANTIMICROBIAL SYNTHETIC BODIES WITH IMPROVED LONG-TERM BEHAVIOR
PROCEDE DE FABRICATION DE CORPS EN PLASTIQUE ANTI-MICROBIENS A COMPORTEMENT AMELIORE SUR LA DUREE

(30) Priorität: 30.07.1999 DE 19936059; 17.03.2000 DE 10013248
(43) Veröffentlichungstag der Anmeldung: 05.06.2002
(60) Teilanmeldung: 04013082.5
(73) Patentinhaber: Guggenbichler, J. Peter, 91054 Erlangen (DE); Hirsch, Andreas, 91054 Erlangen (DE)
(72) Erfinder: Guggenbichler, J. Peter, 91054 Erlangen (DE); Hirsch, Andreas, 91054 Erlangen (DE)
(74) Vertreter: Hofstetter, Schurack & Partner
(86) Internationale Anmeldenummer: PCT/DE2000/002493
(87) Internationale Veröffentlichungsnummer: WO 2001/009229

(56) Entgegenhaltungen:
- EP-B1- 0 711 113
- WO-A1-94/15462
- DE-A- 2 217 399
- DATABASE WPI Section Ch, Week 199523 Derwent Publications Ltd., London, GB; Class A60, AN 1995-175662 XP002152345 & JP 07 097767 A (DAISO CO LTD), 11. April 1995 (1995-04-11)
- DATABASE WPI Section Ch, Week 199936 Derwent Publications Ltd., London, GB; Class A82, AN 1999-425182 XP002152346 & JP 11 172154 A (NIPPON KENKYUSHO KK), 29. Juni 1999 (1999-06-29)
- DATABASE WPI Section Ch, Week 199115 Derwent Publications Ltd., London, GB; Class A97, AN 1991-104975 XP002152347 & JP 03 045709 A (NICHIBI KK), 27. Februar 1991 (1991-02-27)

## Beschreibung

Die Erfindung betrifft Verfahren zur Herstellung von antimikrobiellen metallhaltigen Kunststoffkörpern, insbesondere Gegenstände für den medizinischen Bedarf. Diese Gegenstände werden insbesondere in Form von Kathetern verwendet.

Ein erheblicher Nachteil von Kunststoffgegenständen für den medizinischen Bedarf, insbesondere von Kurz- und Langzeitkathetern, besteht m der leichten Besiedelbarkeit der verwendeten Kunststoffe mit oft multi-resistenten Keimen, die auf der Oberfläche des Kunststoffkörpers, bzw. auf der Katheteraußen- und -innenseite, einen Biofilm bilden. Eine prophylaktische Imprägnierung der Oberflächen mit Antibiotika scheidet wegen der damit verbundenen hohen Selektion von resistenten Mikroorganismen aus.

In den letzten Jahren wurden daher zahlreiche Versuche unternommen, die Kunststoffoberflächen mit Silberionen, die z.B. aus Silbernitrat, -acetat, -chlorid stammen, zu imprägnieren. Silberionen besitzen von allen Schwermetallionen ein sehr breites antimikrobielles Spektrum und eine hohe Toxizität gegenüber Mikroorganismen durch z.B. die Bindung an die Zellwand über SH-Gruppen, Blockierung der Atmungskette, Unterbinden der Zellproliferation durch DNA-Bindung, aber eine geringe Toxizität gegenüber animalischen Zellen. Hierbei konnte jedoch in verschiedenen klinischen Studien keine ausreichende mikrobielle Wirksamkeit beobachtet werden. Zusätzlich führt die ätzende Wirkung bzw. die schlechte Wasserlöslichkeit von Silbersalzen zu weiteren Problemen in der Anwendung.

Bei Kontakt von Metalloberflächen wie z.B. Silber mit physiologischer NaCl-Lösung werden in Abhängigkeit von der Größe der Metalloberfläche Metallionen (Silberionen) in Freiheit gesetzt. Die Beimischung von Metallpulver, z.B. von Silberpulver, zu einem Polymer, z.B. Polyurethan, führt jedoch nicht zum Erfolg, da auf Grund der geringen Oberfläche relativ hohe Konzentrationen an Metallpulver erforderlich sind, was mechanische Probleme im Kunststoffmaterial verursacht. Die für eine antimikrobielle Wirksamkeit erforderliche kritische Oberfläche ist daher durch Zumischen von Metallpulver nicht erreichbar.

EP-A-0 711 113 offenbart eine neue Technologie, bei der metallisches Silber auf Polyurethanfolien aufgedampft und diese in zerkleinerter Form compoundiert werden. Dadurch konnte eine gleichmäßige Verteilung von Silberpartikeln im Polymermaterial erreicht und somit eine für eine bakteriostatische Wirksamkeit ausreichend große Oberfläche erzielt werden. Die antimikrobielle Wirksamkeit dieser Kunststoffkörper ist sowohl hinsichtlich Reduktion und Verhinderung von Adhärenz, Biofilmbildung und Langzeitverhalten als auch Toxizität und Verträglichkeit sehr gut belegt. Die Anwendbarkeit vorstehender Kunststoffkörper wird jedoch durch einen zeitaufwendigen und kostenintensiven Herstellungsprozess, vor allem verursacht durch das Bedampfen mit Silber, limitiert

Ferner beschreibt US-A-5,180,585 eine antimikrobielle Zusammensetzung, umfassend anorganische Teilchen mit einer ersten mikrobiziden Schicht und einer zweiten Schicht zum Schützen der darunterliegenden ersten Schicht. Das Herstellungsverfahren ist relativ komplex.

Die Aufgabe der vorliegenden Erfindung ist somit die Bereitstellung eines Verfahrens zur Herstellung von antimikrobiell wirksamen Kunststoffkörpern, welche die vorstehenden Nachteile nicht aufweisen, d.h. einfach herstellbar sind und eine ausreichende Silberionenkonzentration an der Oberfläche liefern.

Gelöst wird diese Aufgabe durch ein Verfahren, das dadurch gekennzeichnet ist, daß vor dem Formen des Kunststoffkörpers mindestens ein Bestandteil des Vorprodukts des Formkörpers mit einem Metallkolloid behandelt wird, und dem Vorprodukt anorganishe Teilchen zugegeben werden, wobei das Metallkolloid durch Reduktion einer Metallsalzlösung hergestellt wird und zur Stabilisierung des entstehenden Kolloids Schutzstoffe eingesetzt werden.

Als Ausgangsmaterial für den Kunststoffkörper kommen viele polymere Verbindungen in Betracht, die im medizinischen Bereich üblicherweise verwendet werden. Diese sind insbesondere Polyethylen, Polypropylen, vernetzte Polysiloxane, Polyurethane, Polymere auf (Meth)acrlyat-Basis, Cellulose und Cellulosederivate, Polycarbonate, ABS, Tetrafluorethylenpolymere und Polyethylenterephthalate, sowie die entsprechenden Copolymere. Besonders bevorzugt sind Polyurethan, Polyethylen und Polypropylen sowie Polyethylen-Polypropylen-Copolymere. Das eingesetzte Metall ist vorzugsweise Silber, Kupfer, Gold, Zink oder Cer. Von diesen Metallen ist Silber besonders bevorzugt.

Neben dem kolloidalen Metall wird bei der Herstellung der erfindungsgemäßen Kunststoffkörper ein oder mehrere Polymerwerkstoffe eingesetzt. Dem Gemisch aus kolloidalem Metall und Kunststoff(en) können auch weitere Additive zugesetzt werden. Dies sind insbesondere anorganische Teilchen wie Bariumsulfat, Calciumsulfat, Strontiumsulfat, Titanoxid, Aluminiumoxid, Siliziumoxid, Zeolithe, Glimmer, Talk, Kaolin usw. Besonders bevorzugt ist dabei Bariumsulfat, das gleichzeitig als Röntgenkontrastmittel für besondere Anwendungsformen dienen kann.

Vor dem Formen wird ein oder mehrere Polymerbestandteile mit der kolloidalen Metallösung behandelt und/oder ein oder mehrere der anorganischen Additive.

Nach dem Mischen der (teilweise) mit einem kolloidalen Metall behandelten Ausgangsstoffe wird das erhaltene Gemisch weiterverarbeitet, um einen Kunststofformling zu erhalten. Dies kann in Mischern, Knetern, Extrudern, Spritzgießmaschinen oder (Heiß)pressen geschehen.

Die Metallkolloide, mit denen die Kunststoffe oder anorganischen Teilchen behandelt werden, stellt man durch Reduktion von Metallsalzlösungen her. Zur Stabilisierung des entstehenden Kolloids werden Schutzstoffe wie Gelatine, Kieselsäure oder Stärke eingesetzt werden.

Im Falle des bevorzugten Metalls Silber wird z.B. ammoniakalische Silbernitratlösung in der Gelatine langsam mit einem geeigneten Reduktionsmittel versetzt. Als Reduktionsmittel können neben Aldehyden (z.B. Acetaldehyd) auch Aldosen (z.B. Glucose), Chinone (z.B. Hydrochinon), anorganische komplexe Hydride (Natrium- oder Caliumboranat), reduzierende Stickstoffverbindungen (Hydrazin, Polyethylenimin) sowie Ascorbinsäure verwendet werden.

Kunststoffvorprodukte, wie z.B. Pellets, und/oder die anorganischen Teilchen, wie z.B. Bariumsulfat, werden dann mit dieser kolloidalen Silberlösung behandelt, getrocknet und in die entsprechende Form gebracht. Das Aufbringen des Silberkolloids auf die Ausgangsstoffe und das anschließende Trocknen kann mehrmals wiederholt werden, so daß auf diesem Wege sehr hohe Silberkonzentrationen in das Kunststoffmaterial eingebracht werden können. Dies ist insbesondere bei der Silberbeschichtung von Bariumsulfat vorteilhaft, da so eine vorherige Beschichtung der Kunststoff-Pellets nicht zwingend erforderlich ist.

Die Suspension kann auch durch Filtration vom Lösungsmittel und anschließend durch Nachwaschen zunächst mit ca. 5 %-iger Ammoniaklösung und dann mehrmals mit aqua dest. von allen niedermolekularen organischen Verbindungen befreit werden. Der Filterrückstand liefert wie vorstehend nach Lufttrocknung ein homogenes Material. Auch dieser Prozess kann mehrmals wiederholt werden.

Durch Variation der Kolloidstabilisatoren sowie durch Variation oder Weglassen der Reduktionsmittel kann die Partikelgröße des Silbers und damit die Mobilität der entstehenden Silberionen in einem weiten Bereich gesteuert und darüber hinaus durch die Verwendung von niedermolekularen Aldehyden als Reduktionsmittel, welche die Gelatine teilweise vernetzen, eine sehr gute Haftung auf dem Polymer erreicht werden.

Im folgenden wird das erfindungsgemäße Verfahren durch Beispiele veranschaulicht.

### Beispiel 1:

### Herstellung des Silberkolloids

1,0 g Gelatine (DAB) werden bei 40 °C in 100 ml aqua dest. unter Rühren gelöst. Hierzu gibt man anschließend 1,0 g (5,88 mmol) AgNO₃ p.a. und versetzt die entstandene Lösung mit 1,0 ml (14,71 mmol) 25 %-igen NH₃-Wasser.
Zur Darstellung des Silberkolloids werden zu vorstehender Lösung bei 40 °C langsam über einen Zeitraum von 30 min 258,7 mg (5,88 mmol, 330 µl) Acetaldehyd gelöst in 50 ml aqua dest. getropft.

### Beispiel 2:

### Beschichtung von Polyurethan-Pellets

10 min nach beendetem Zutropfen gemäß Beispiel 1 werden ca. 50 g Polyurethan-Pellets aus Tecothane TT- 1085A zugesetzt und zur Beschichtung mit kolloidalem Silber zunächst 2 h bei 40 °C und anschließend 3 h bei RT kräftig gerührt.
Man trennt das Silberkolloid durch rasche Filtration über einen Faltenfilter geeigneter Porengröße ab, wäscht die Pellets noch einmal mit dem Filtrat nach und überführt die noch feuchten Pellets in eine Abdampfschale. Nach dem Entfernen überschüssiger nicht am Polymer anhaftender Silberkolloidlösung wird 10 h bei 70 °C getrocknet.

### Beispiel 3:

### Adsorption von kolloidalem Silber an Bariumsulfat

a) In 500 ml aqua dest. werden bei 50 °C nacheinander 0,666 g Gelatine und anschließend 6,66 g AgNO₃ gelöst. Man versetzt mit ca. 8,5 ml 25 %-iger wässriger NH₃-Lösung bis zur schwach alkalischen Reaktion.
   Hierzu wird langsam bei 50 °C unter kräftigem Rühren eine Lösung aus 3,53 g wasserfreier α-D-Glucose in 150 ml aqua dest. getropft und das entstandene Silberkolloid, wenn ca. die Hälfte der Glucoselösung zugetropft ist, mit 333 g BaSO₄ versetzt. Nach beendetem Zutropfen wird die Suspension noch ca. 2 h bei 50 °C weiterturbiniert und anschließend durch Eindampfen und Trocknen bei 70 °C von den flüchtigen Anteilen befreit. Die Zerkleinerung des Materials erfolgt in einem Handmörser.
b) Vorgehensweise analog Beispiel 3a), jedoch unter Verwendung von 6,66 g pyrogener Kieselsäure (Degussa, Aerosil 200) anstelle von Gelatine. Die Teilchengröße des kolloidalen Silbers lag im Bereich von 10 bis 50 nm, bestimmt mittels einer REM-Aufnahme.

### Beispiel 4:

### Alternative Adsorption von kolloidalem Silber an Bariumsulfat

Verfahrensweise analog Beispiel 3a), jedoch mit 1,2 1 aqua dest., 2 g Gelatine, 20 g AgNO₃ und 26 ml 25 %-iger NH₃-Lösung. Als Reduktionsmittel wird eine Lösung aus 10,59 g Glucose in 400 ml aqua dest. verwendet und analog Beispiel 3a) mit 333 g BaSO₄ versetzt. Die Suspension wird anschließend 3 h bei 50 °C weiterturbiniert und zur Vervollständigung der Reaktion ca. 8 h bei 70 °C gehalten. Das an BaSO₄ absorbierte Ag-Kolloid wird durch Filtration der noch möglichst warmen Suspension und anschließendem viermaligem Nachwaschen des Rückstandes mit aqua dest. vom Wasser und den darin löslichen Bestandteilen (Gelatine, Gluconsäure, NH₄NO₃ und NH₃) abgetrennt. Die Trocknung erfolgt bei 70 °C und die Zerkleinerung wie in Beispiel 3a).

Der Restanteil an organischem Material (Gelatine, Gluconsäure, Glucose) des nach Beispiel 4 erhaltenen Materials wurde unter der Voraussetzung, daß Gelatine und Gluconsäure unter den verwendeten Bedingungen eine vergleichbare Löslichkeit in Wasser besitzen, mittels zweier voneinander unabhängigen Methoden bestimmt.

### Durch Verbrennungsanalyse:

Hierbei liegen die C- und H-Werte unterhalb der vom Gerätehersteller angegebenen Meßtoleranz von 0,3 %, d.h. daß ein fertiges compoundiertes Polyurethanmaterial mit 20 % BaSO₄ und 0,8 % Ag rechnerisch einen organischen Gesamtrestanteil von theoretisch maximal 0,182 Gew.-% (maximal mögliche untere Erfassungsgrenze des Geräts) besitzt. Der tatsächliche Wert dürfte somit wesentlich geringer sein.

### Durch Thermogravimetrie:

Durch Vergleich des nach Beispiel 4 erhaltenen Materials mit einer identisch hergestellten, aber nicht ausgewaschenen Referenzprobe (Gewichtsverlust ca. 3,2 %) und reinem BaSO₄ ergibt sich ein Gesamtgewichtsverlust von maximal 0,28 Gew.-% (Gelatine: 0,045 Gew.-%, Gluconsäure: 0,235 Gew.-%) oder besser. Das fertige compoundierte Polyurethan mit 20 % BaSO₄ und 0,8 % Ag weist somit einen organischen Gesamtrestanteil von≤ 0,056 Gew.-% (Gelatine: ≤ 0,009 Gew.-%, Gluconsäure: ≤ 0,047 Gew.-%) auf Hierbei ist wegen der bedeutend größeren Empfindlichkeit die Thermogravimetrie der Verbrennungsanalyse vorzuziehen.

### Beispiel 5:

### Bestimmung der antibakteriellen Wirksamkeit

Zur Bestimmung der Besiedelbarkeit der erfindungsgemäßen Kunststoffkörper mit Keimen wurden jeweils fünf zylinderförmige Proben des entsprechenden Kunststoffs (Durchmesser 3 mm, Länge 13 mm) mit einer Zusammensetzung enthaltend Staphylococcus epidermis in einer Trypcase-Soy-Broth-Nährlösung bei 175 °C inkubiert. Folgende Kunststoffkörper wurden untersucht (Nummer 1 ist handelsüblich und unbehandelt, Nummer 2 und 3 sind erfindungsgemäß):
Prüfkörper 1: Stück aus einem PU-Katheter der Firma Arrow (ES 04701)
Prüfkörper 2: gemäß Beispiel 2 der vorliegenden Erfindung
Prüfkörper 3: gemäß Beispiel 3 der vorliegenden Erfindung.

Die jeweils 5 Prüfkörper wurden vier Testreihen unter folgenden Bedingungen unterzogen:
- Testreihe 1:: anfängliche Konzentration von Staphylococcus epidermis 5 x 10⁷ CFU/ml
- Testreihe 2:: anfängliche Konzentration von Staphylococcus epidermis 10⁸ CFU/ml
- Testreihe 3:: wie Testreihe 1, jedoch nach einer 5-stündigen Vorinkubation in physiologischer Pufferlösung bei 37 °C gemessen
- Testreihe 4:: wie Testreihe 1, wobei die Kunststoffkörper mit steril filtriertem natürlichen Harn bei 37 °C 4 Stunden lang vorbehandelt wurden.

Tabelle 1 zeigt die Anzahl der besiedelten Kunststoffkörper. Bestimmt wurde dies durch visuelle Kontrolle.

**Tabelle 1**

| | Prüfkörper-Typ | Zahl der besiedelten Prüfkörper | | | |
|---|---|---|---|---|---|
| | | Testreihe 1 | Testreihe 2 | Testreihe 3 | Testreihe 4 |
| Vergleich | 1 | 3 | 5 | 4 | 5 |
| Erfindung | 2 | 0 | 0 | 0 | 0 |
| | 3 | 0 | 3 | 0 | 0 |

Die Kathetermaterialien zeigen nach dem Compoundieren keine Beeinträchtigung der für therapeutische Zwecke nötigen mechanischen Eigenschaften (Rauhigkeit, Homogenität und Elastizität). Hierbei kann das Verfahren gut an wechselnde Anforderungen im Produktionsprozess angepaßt werden, da die antimikrobielle Wirksamkeit unabhängig davon erhalten bleibt, ob das Silber über eine Beschichtung der Polyurethan-Pellets (Beispiel 2) oder über das Röntgenkontrastmittel (Beispiel 3 und 4) in das Polymermaterial eingebracht wird.
Die erfindungsgemäßen Kunststoffgegenstände zeigen bei vergleichbar geringer Toxizität eine signifikant höhere antimikrobielle Wirksamkeit hinsichtlich Adhärenz und Biofilmbildung sowie ein deutlich verbessertes Langzeitverhalten als bisherige Werkstoffe.
Die erfindungsgemäßen Herstellungsverfahren sind gut kontrollierbar, kostengünstig und für eine Produktion im größeren Maßstab geeignet. Zusätzlich wurde mit Beispiel 4 ein Verfahren ausgearbeitet, alle "Hilfschemikalien" aus dem anorganischen Kontrastmittel zu entfernen, so daß eine Zertifizierung des Verfahrens keine Probleme bereiten sollte.

### Beispiel 6:

### Zeitabhängigkeit der antimikrobiellen Wirksamkeit

Katheter (die Mengenangaben sind auf das fertige, compoundierte Material bezogen):

| | | |
|---|---|---|
| 1) | Polyurethankatheter | 20 % BaSO₄ + 0,8 % Ag 1,0 cm Länge (Beispiel 4) |
| 2a) | Silikonkatheter | 25 % BaSO₄ + 1 % Ag 1 cm Länge, 1,3 mm Dicke und 2 mm Breite (Beispiel 4) |
| 2b) | Silikonkatheter | 25 % BaSO₄ + 0,33 % Ag + 0,33 % SiO₂ (Beispiel 3b) Wandstücke von Silikon 1 cm Länge, 1 mm Dicke und 2 mm Breite |
| 3) | Kontrolle: | ArgenTec 1 Lumenkatheter (Sicuris) Extr. 1/99 20 % BaSO₄ + 0,9-1 % Ag |

Sterilisation: Aufbewahrung im Heißluftschrank bei 90 °C über 3 Stunden.
Vorangegangene Untersuchungen zeigten die Keimfreiheit der Proben nach dieser Zeit. (Proben sind weitgehend vorher bereits nicht keimbesiedelt)
Keime: S. epidermidis (Ref.: Infection Suppl. 6/99)
E. coli "
Nährmedium: Trypcase Soja
Vorgangsweise:
- Proben werden 8 Stunden lang in einer Suspension NaCl 0,45 % mit 2,5 % Glukose mit 5 x 10⁷ Keimen bei Zimmertemperatur inkubiert
- Anschließend wird die Keimsuspension abzentrifugiert
- 2 x Waschen (2 min Resuspension in physiologischer Kochsalzlösung unter Schwenken)
- Transferierung der Proben in sterile Kochsalzösung in einer Petrischale
- Stündliche, nach 6 Stunden 2-stündliche Entnahme einer Probe und Transferierung nach leichtem Schwenken in physiologischer Kochsalzlösung in Trypcase Soja Medium
- Inkubation für 24-36 Stunden
- Beurteilung auf Sterilität der Probe (Trübung = Endpunktmessung).

### Ergebnisse der Untersuchungen mit S. epidermidis

Alle Proben werden 5-fach (+++++) getestet

| Zeit: | Probe 1 | Probe 2 a | Probe 2 b | Kontrolle |
|---|---|---|---|---|
| h | | | | |
| 0 | +++++ | +++++ | +++++ | +++++ |
| 1 | +++++ | +++++ | +++++ | +++++ |
| 2 | +++++ | +++++ | +++++ | +++++ |
| 3 | ++++- | ++++- | +++++ | +++++ |
| 4 | ++++- | ++++- | +++++ | +++++ |
| 5 | ++--- | ++--- | ++++- | +++++ |
| 6 | ----- | +---- | ++--- | +++++ |
| 8 | ----- | ----- | ----- | +++++ |
| 10 | ----- | ----- | ----- | +++++ |
| 12 | ----- | ---- | ----- | ++++- |
| 16 | ----- | ----- | ----- | +++-- |
| 18 | ----- | ----- | ----- | +++-- |
| + = Bouillone nach 36 Stunden trüb | | | | |
| - = Bouillone nach 36 Stunden klar (steril) | | | | |

### Diskussion:

In dieser Untersuchung konnte eine zeitaufgelöste antimikrobielle Wirksamkeit von Festkörpern geprüft werden. Es zeigt sich, daß die antimikrobielle Wirksamkeit der silbergefüllten Proben bereits nach 6 Stunden besteht und ein kontaminierter Katheter in dieser Zeit auch bei einem unphysiologisch hohen Inokulum bereits wiederum steril ist. Eine geringere Ag-Konzentration wie bei Probe 2b führt ebenfalls zu einem positiven Ergebnis.

### Ergebnisse der Untersuchungen mit E. coli

Alle Proben werden 5-fach (+++++) getestet

| Zeit: | Probe 1 | Probe 2 a | Probe 2 b | Kontrolle |
|---|---|---|---|---|
| h | | | | |
| 0 | | +++++ | +++++ | +++++ |
| 1 | | +++++ | +++++ | +++++ |
| 2 | | +++++ | +++++ | +++++ |
| 3 | | ++++- | +++++ | +++++ |
| 4 | | ++++- | +++++ | +++++ |
| 5 | | ++++- | ++++- | +++++ |
| 6 | | +++-- | ++++- | +++++ |
| 8 | | ++--- | +++-- | +++++ |
| 10 | | +--- | +---- | +++++ |
| 12 | | ----- | ----- | +++++ |
| 16 | | ----- | ----- | ++++- |
| 18 | | ----- | ----- | ++++- |

Die Erebnisse für S. epidermidis sind auch nach 1,2 und 3 Wochen Elution des Silbers in physiologischer NaCl-Lösung gleich gut und zeigen identische Ergebnisse wie in Tabelle 1.

Die Untersuchung der Cytotoxizität wurde durch die Firma Toxikon, Bedford Mass, USA durchgeführt. Es zeigte sich, daß die hergestellten Proben nicht toxisch sind und den Erfordernissen des Elutions-Testes ISO 10993 entsprechen.

## Patentansprüche

1. Verfahren zur Herstellung eines antimikrobiellen Kunststoffkörpers, umfassend das Formen eines Vorprodukts, wobei dem Vorprodukt anorganische Teilchen zugegeben werden, **dadurch gekennzeichnet, daß** vor dem Formen mindestens ein Bestandteil des Vorproduktes mit einem Metallkolloid behandelt wird, wobei das Metallkolloid durch Reduktion einer Metallsalzlösung hergestellt wird, und zur Stabilisierung des entstehenden Kolloids Schutzstoffe eingesetzt werden.

2. Verfanren nach Anspruch 1, wobei das Vorprodukt aus einem oder mehreren polymeren Materialien besteht.

3. Verfahren nach Anspruch 2, wobei das Vorprodukt aus Polyurethan besteht.

4. Verfahren nach einem der Ansprüche 1 bis 3, wobei dem Kunststoffvorprodukt neben den polymeren Werkstoffen weitere Additive zugegeben werden.

5. Verfahren nach Anspruch 4, wobei die anorganischen Teilchen Bariumsulfat, Calciumsulfat, Strontiumsulfat, Titanoxid, Aluminiumoxid, Siliziumoxid, Zeolithe, Glimmer, Talk oder Kaolin umfassen.

6. Verfahren nach Anspruch 5, wobei die anorganischen Teilchen Bariumsulfat und/oder pyrogene Kieselsäure umfassen.

7. Verfahren nach einem der Ansprüche 1 bis 6, wobei eines oder mehrere der Vorproduktbestandteite mit dem kolloiden Metall behandelt werden.

8. Verfahren nach einem der Ansprüche 4 bis 6, wobei sowohl der Kunststoff als auch die anorganischen Teilchen mit dem koliolden Metall behandelt werden.

9. Verfahren nach einem der Ansprüche 4 bis 6, wobei die anorganischen Teilchen mit dem Metallkolloid behandelt werden.

10. Verfahren nach einem der Ansprüche 1 bis 9, wobei das Metallkolloid kolloidales Silber ist.

11. Verfahren nach einem der Ansprüche 1 bis 10, wobei das behandelte Vorprodukt durch Mischen, Kneten, Extrudieren, Spritzgießen oder (Heiß)pressen in die endgültige Form gebracht wird.

## Claims

1. Method for manufacturing an antimicrobial plastics body comprising the moulding of an intermediate product, wherein inorganic particles are added to the intermediate product, **characterised in that** before the moulding at least one component of the intermediate product is treated with a metal colloid, wherein the metal colloid is prepared by reduction from a metal salt solution and wherein for stabilizing the forming colloid protective agents are used.

2. Method according to claim 1, whereby the intermediate product comprises one or more polymer materials.

3. Method according to claim 2, whereby the intermediate product consists of polyurethane.

4. Method according to one of the claims 1 to 3, whereby further additives are added to the plastics intermediate product apart from the polymer materials.

5. Method according to claim 4, whereby the inorganic particles include barium sulphate, calcium sulphate, strontium sulphate, titanium oxide, aluminium oxide, silicon oxide, zeolites, mica, talcum or kaolin.

6. Method according to claim 5, whereby the inorganic particles include barium sulphate and/or pyrogenic silicic acid.

7. Method according to one of the claims 1 to 6, whereby one or more of the intermediate product components are treated with the colloidal metal.

8. Method according to one of the claims 4 to 6, whereby both the plastics and the inorganic particles are treated with the colloidal metal.

9. Method according to one of the claims 4 to 6, whereby the inorganic parties are treated with the metal colloid.

10. Method according to one of the claims 1 to 9, whereby the metal colloid is colloidal silver.

11. Method according to one of the claims 1 to 10, whereby the treated intermediate product is brought into its final form by mixing, kneading, extruding, injection moulding or (hot) pressing.

## Revendications

1. Procédé de fabrication d'un corps en matière plastique antimicrobien comportant le moulage d'un pré-produit, des particules inorganiques étant ajoutées au pré-produit, **caractérisé en ce qu'**avant le moulage, on traite au moins une composante du pré-produit avec un colloïde métallique, le colloïde métallique étant obtenu par réduction d'une solution de sel(s) métallique(s) et **en ce qu'**on emploie des substances protectrices pour stabiliser le colloïde qui se forme.

2. Procédé selon la revendication 1, dans lequel le pré-produit se compose d'un ou de plusieurs matériaux polymères.

3. Procédé selon la revendication 2, dans lequel le pré-produit est composé de polyuréthane.

4. Procédé selon l'une des revendications 1 à 3, dans lequel on ajoute au pré-produit plastique, en plus des matériaux polymères, d'autres additifs.

5. Procédé selon la revendication 4, dans lequel les particules inorganiques comprennent du sulfate de baryum, du sulfate de calcium, du sulfate de strontium, de l'oxyde de titane, de l'oxyde d'aluminium, de l'oxyde de silicium, des zéolithes, du mica, du talc ou du kaolin.

6. Procédé selon la revendication 5, dans lequel les particules inorganiques comprennent du sulfate de baryum et/ou de l'acide silicique pyrogène.

7. Procédé selon l'une des revendications 1 à 6, dans lequel un ou plusieurs des composantes du pré-produit sont traitées avec le métal colloïdal.

8. Procédé selon l'une des revendications 4 à 6, dans lequel tant le matériau plastique que les particules inorganiques sont traités avec le métal colloidal.

9. Procédé selon l'une des revendications 4 à 6, dans lequel les particules inorganiques sont traitées avec le colloïde métallique.

10. Procédé selon l'une des revendications 1 à 9, dans lequel le colloïde métallique est de l'argent colloïdal.

11. Procédé selon l'une des revendications 1 à 10, dans lequel le pré-produit traité est amené dans la forme finale par mélange, malaxage, extrusion, moulage par injection ou pressage (à chaud).
